# EUROPEAN PATENT APPLICATION

(11) **EP 1 967 219 A2**
(43) Date of publication of application: **10.09.2008**
(21) Application number: 07253803.6
(22) Date of filing: 26.09.2007
(51) Int. Cl.: A61L 27/14, A61L 27/56, A61L 27/58

(54) **Tissue growth devices**

(30) Priority: 29.09.2006 US 848048
(71) Applicant: Johnson & Johnson Regenerative Therapeutics, LLC, Raynham, MA 02767 (US)
(72) Inventor: Keeley, Daniel, Boston-ma 02215 (US); Shetty, Dhanuraj, Somerset -NJ 08873 (US); Hammer, Joseph J, NJ 08844 (US)
(74) Representative: Belcher, Simon James

(57) **Abstract**

The invention relates generally to devices for organ replacement and regenerative medicine providing a biocompatible and biodegradable scaffold capable of integral cell growth that forms a hollow chamber, as well as methods for producing such devices by melt-blowing a web of flexible, polymer fibres in the presence of a porogen to produce a seamless, three-dimensional shape.

## Description

The present invention relates to devices and methods for organ replacement and regenerative medicine. More specifically, the present invention provides for a hollow chamber formed by a biocompatible and biodegradable scaffold capable of integral cell growth that may facilitate the regeneration of an organ.

Regenerative medicine is a developing field targeted at treating disease and restoring human tissues. Potential therapies may prompt the body to autonomously regenerate damaged tissue. Additionally, tissue engineered implants may also prompt regeneration. Developing approaches may also enable direct transplantation of healthy tissues into a damaged-tissue environment.

Many of these new therapies may require implantable biocompatible and biodegradable scaffolds for use both in vitro and in vivo. These scaffolds may augment healing through tissue infiltration or by providing suitable means of cell attachment and proliferation. Hollow chambers comprising biocompatible and biodegradable scaffolds are unique in that they may have the ability not only to replace damaged tissue but to replace entire organs. During 2001, at least 80,000 persons awaited organ transplants, but less than 13,000 transplants were made available. Hence, there remains a huge unmet need for appropriate biocompatible and biodegradable scaffolds upon which entire human organs or tissues can grow or regenerate.

Biocompatible scaffold fabrication methods are challenged in their ability to produce effective scaffolds from a limited number of materials. At the moment, one of the greatest challenges lies in producing a mechanically stable scaffold with high enough porosity to augment healing through cell proliferation and tissue ingrowth. There is also a lack of adequate methodologies to make these scaffolds into hollow structures. These and other deficiencies in the prior art are overcome by the present invention.

The present invention provides a tissue growth device for organ replacement and regenerative medicine comprising a biocompatible, biodegradable scaffold capable of integral cell growth that forms a hollow chamber.

The device of the invention can be produced by melt-blowing a web of flexible polymer fibres in the presence of a porogen. An appropriate melt-blowing technique might include distributing molten polymer resin onto a rotating collapsible object to create a seamless, three-dimensional shape.

In another aspect, the invention provides a tissue growth device for organ replacement and regenerative medicine that includes biological factors, such as growth factors, hormones and cytokines, or drugs, such as antibiotics, analgesics and anti-inflammatory agents, or combinations thereof.

In a further aspect, the invention provides a tissue generated by a growth device for organ replacement and regenerative medicine.

Embodiments of the invention are described below by way of example with reference to the accompanying drawings, in which:
Figure 1 depicts an embodiment of the present invention in which melt blowing technology is used to manufacture fibrous webs from molten polymer resin extruded through spinnerettes by high-velocity air.
Figure 2 depicts an embodiment of the present invention in which spinnerettes are aimed at a take-up surface on which the polymer web is formed.
Figure 3 depicts an embodiment of the present invention in which a rotating collapsible object is used to create seamless, three-dimensional shapes of polymer web.
Figures 4 and 5 depict an embodiment of the present invention in which a porogen is added during the fabrication of the non-woven web.

Other than in the operating examples, or where otherwise indicated, all numbers expressing quantities of ingredients or reaction conditions used herein should be understood as modified in all instances by the term "about." The term "about" when used in connection with percentages may mean ± 1%.

The present invention provides for a device for organ replacement and regenerative medicine comprising a biocompatible, biodegradable scaffold capable of integral cell growth that forms a hollow chamber. The scaffold may also act as a substrate or carrier for cells, growth factors, bioactives, and drugs.

Regarding the "hollow chamber," the device may consist of a single chamber that is hollow or substantially hollow. Alternatively, the device may consist of more than one chamber that is hollow or substantially hollow. The chambers may or may not be attached to each other. Indeed, the invention contemplates an aggregate of individual hollow chambers as well as a subdivided single chamber. "Integral cell growth" refers to the process including, but not limited to, cell attachment, proliferation, differentiation, infiltration, residence, and outgrowth such that as the scaffold degrades, tissue growth and organ regeneration may give rise to a biologically functioning tissue or organ.

US-5609809 refers to the use of melt blown technology for "general disposable-type household supplies such as sanitary materials, wiping cloths, and packaging materials" that while biodegradable, are not meant for medical applications. US-A-2004/0202700 discloses "a method for making an infection-preventative fabric article which is suitable for a non-invasive or topical usage as a medical treatment fabric". These fabrics are used during surgery rather than for use in implant technologies. US-5108428 and US-A-2004/0078004 both refer to melt-blowing technology as a technique to manufacture a component of a medical implant or device but specifically, as a means to create an area of implant that will serve to anchor it to the body. US-A-2001/0010022 refers to the hypothetical use of this technology in a tracheal prosthesis, external ear prosthesis, and liver reactor. Although the document discusses using melt-blown technology to create a "three dimensional shaped article", porogens are not incorporated into the process. US-6913762 specifically refers to using the technology in coating stents "implantable within the vascular system of a mammal". US-5783504 is for a composite structure of a "homopolymer or copolymer of lactic acid and at least one ply of film of thermoplastic homopolymer of biodegradable aliphatic polymer" whereby melt-blowing is only used in a single area for anchoring a larger implanted device. The technology is not utilized to fully manufacture the implant. US-A-2004/026600 discloses "a biocompatible scaffold for tissue culture and cell culture and for producing implants or implant materials and fibres that are electrostatically flocked onto at least one side of at least one base material". US-A-2005/0251083 discloses "a biointerface membrane" that is "adapted to support tissue ingrowth and to interfere with barrier cell layer formation" whereby melt-blowing is suggested as a method of manufacture. US-6165217 discloses "an article comprising melt-formed continuous filaments intermingled to form a porous web wherein said filaments are self-cohered to each other at multiple contact points, wherein said filaments comprise at least one semi-crystalline polymeric component covalently bonded to or blended with at least one amorphous component".

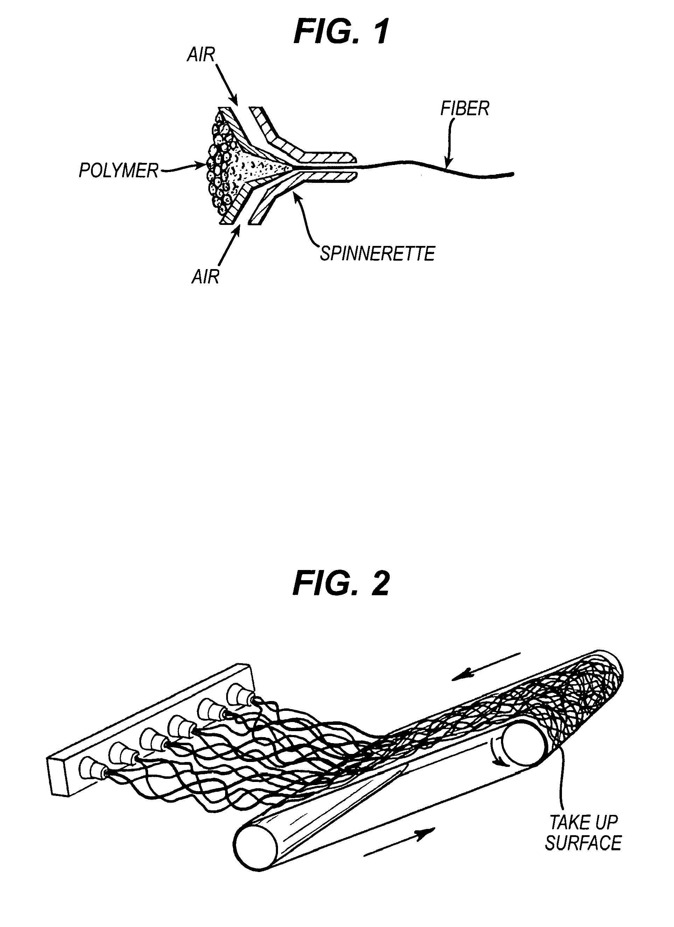

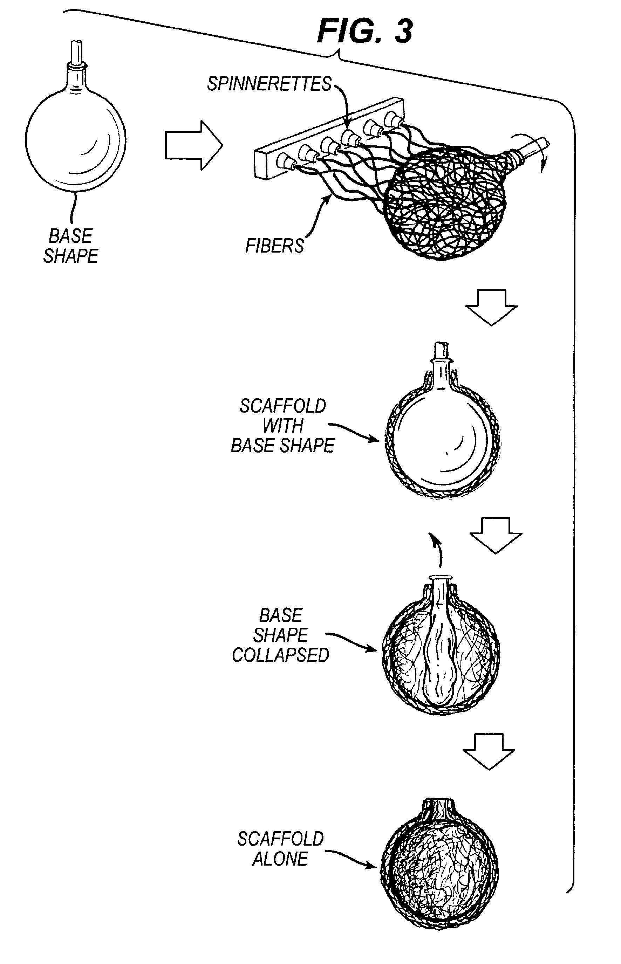

Melt-blowing technology can be utilized to manufacture fibrous webs from molten polymer resin extruded from spinnerettes onto a rotating collapsible object in the presence of a porogen. See Figures 1 and 2. The collapsible object can be made to rotate or otherwise move therefore allowing a coating of extruded polymer to layer itself substantially evenly on a conveyor belt of solid object. Continuous rotation of the surface will produce an increasingly thick or dense layer due to more polymer being deposited. The use of a collapsible object creates seamless, three-dimensional shapes of polymer web. Specifically, the final product may be a hollow shape with a single outlet from which the collapsed shape has been removed. See Figure 3. More complex geometries may be achieved by using suitably shaped tooling such as a mould or mandrel to guide the formation of the melt-blown filaments into a specific shape.

Melt-blown technology is able to incorporate synthetic biopolymers, such as PGA, PLA or their respective copolymers, and natural polymers. A scaffold constructed of either material is both biocompatible and resorbable but may not be sufficiently porous to facilitate optimal proliferation of cells or advanced tissue ingrowth. To overcome this obstacle, a porogen may be added during the fabrication of the non-woven web. Porogens such as salt or glucose spheres can be dusted or blown onto the molten fibres during their extrusion. Gelatin microspheres can also be used. The resulting scaffold's porosity can be controlled by the amount of porogen added, while the pore size is dependent on the size of the spheres. As these particles enter the turbulent air, they are randomly incorporated into the web. Because the filaments in the melt-blown structure will typically shrink due to crystallization as they age, the porous structure may undergo an annealing process with the porogen material in place. Once the porogen-fibre composite is annealed, the entire construct may then be submerged in water so that the porogens dissolve or leach out of the web. The resulting matrix contains polymer fibres but with increased distance between them to effect porosities. In one embodiment, the matrix has more porogen and hence, more porosity, the porosity in excess of 90%.

In various embodiments of the present invention, the polymers or polymer blends that are used to form the biocompatible, biodegradable scaffold may contain pharmaceutical compositions. The previously described polymer may be mixed with one or more pharmaceuticals prior to forming the scaffold. Alternatively, such pharmaceutical compositions may coat the scaffold after it is formed. The variety of pharmaceuticals that can be used in conjunction with the scaffolds of the present invention includes any known in the art. In general, pharmaceuticals that may be administered via the compositions of the invention include, without limitation: anti-infectives such as antibiotics and antiviral agents; chemotherapeutic agents; anti-rejection agents; analgesics and analgesic combinations; anti-inflammatory agents; hormones such as steroids; growth factors; and other naturally derived or genetically engineered (recombinant) proteins, polysaccharides, glycoproteins, or lipoproteins.

Scaffolds containing these materials may be formulated by mixing one or more agents with the polymer used to make the scaffold or with the solvent or with the polymer-solvent mixture. Alternatively, an agent could be coated onto the scaffold, preferably with a pharmaceutically acceptable carrier. Any pharmaceutical carrier may be used that does not substantially degrade the scaffold. The pharmaceutical agents may be present as a liquid, a finely divided solid, or any other appropriate physical form. Typically, but optionally, they will include one or more additives, such as diluents, carriers, excipients, stabilizers or the like. In addition, various biologic compounds such as antibodies, cellular adhesion factors, and the like, may be used to contact and/or bind delivery agents of choice (such as pharmaceuticals and other biological factors) to the scaffold of the present invention.

The hollow chamber of the present invention may be useful in regenerating such organs as the bladder whereby the present invention is seeded or engrafted with cells, preferably those of the host. For example, primary rabbit urothelial cells (RUC) have been found to attach readily to unwoven polyglycolic acid polymers in vitro, survive, and grow in vivo (US-5851833). Some differentiated cell types, such as chondrocytes and hepatocytes, have been found to remain functionally differentiated and in some cases to expand in vivo on non-woven polyglycolic acid or polylactic acid polymers. The polymer fibres provide sites for cell attachment, the reticular nature of the polymer lattice allows for gas exchange to occur over considerably less than limiting distances, and the polymers evoke host cell responses, such as angiogenesis which promote cell growth.

Synthetic polymers can also be modified in vitro before use, and can carry growth factors and other physiologic agents such as peptide and steroid hormones, which promote proliferation and differentiation. The polyglycolic acid polymer undergoes biodegradation over a four month period; therefore as a cell delivery vehicle it permits the gross form of the tissue structure to be reconstituted in vitro before implantation with subsequent replacement of the polymer by an expanding population of engrafted cells.

To regenerate such organs as the bladder, the hollow chamber of the present invention may also be implanted without having cells seeded beforehand. The matrix may contain pharmaceuticals or proteins, e.g., antibodies attached to cell adhesion factors that promote cell attachment, proliferation, differentiation, infiltration, residence, and outgrowth such that once the scaffold degrades, a biologically functioning tissue or organ remains.

### EXAMPLES

### Example 1. Melt-Blown Methodology

Melt blown extruder utilizing 0.508 mm (20 mil) 127 mm (5 inch) die; ensure all connections are made to melt blowing apparatus such as electrical and pressure connections; power on melt blowing apparatus. Set each temperature zones to the following conditions

| Zone | Temp °F | Temp °C |
|---|---|---|
| 1 | 450 | 232 |
| 2 | 477 | 247 |
| 3 | 477 | 247 |
| Die | 493 | 256 |

Allow each zone to preheat to specified conditions; remove 90:10 polylactide (inherent viscosity - 1.26DL/g) from container; pour desired amount of polymer into open hopper; close hopper and purge with nitrogen gas. Once closed set machine to 8% throughput and turn on die pressure; maintain a minimum die pressure of 1.38 MPa (200 psi).

A preparation of 90:10 polylactide may be manufactured with varying inherent viscosities. A lower viscosity would allow extrusion at lower heat settings. This in turn may also cause changes in throughput and pressure settings. As a result each polymer should be tested individually. To prevent damage to the polymer, 90:10 polylactide formula should not be heated higher than 260°C (500°F).

An initial formulation of 90:10 polylactide with an inherent viscosity of 1.76 dL.g⁻¹ was tested using the apparatus above. It could only be extruded at 1.38 MPa (200 psi) under the following temperatures.

| Zone | Temp °F | Temp °C |
|---|---|---|
| 1 | 390 | 199 |
| 2 | 490 | 254 |
| 3 | 523 | 273 |
| Die | 525 | 274 |

These temperatures were too high to maintain polymer stability. This could be observed as the extruded polymer was charred and burned. Lowering the temperature to avoid charring prevented fibre formation.

### Example 2. Porogen dispersion methodology

Porogen can be quantitatively added to the extrusion technique utilizing a bulk feeder to qualitatively add mass to the extrusion process. This porogen may be dusted or poured into the extruded polymer stream. Porogen size should be less than 300 µm, to prevent excessive porosity, but large enough to be taken up by the extrusion. The actual size will vary with extrusion velocity and extruded polymer diameter. The stream of particulate must be positioned above the extruded polymer and have a dispersion distance wide enough to encompass the entire polymer stream. Control of the mass flow of material into the extruded stream will result in more or less porous scaffolds.

The take up surface may be flat or three-dimensional. A three-dimensional take up surface may or may not need to be collapsible depending on the required geometry. Increasing and decreasing the speed of the rotated shape will change the spacing and alignment of fibres. One may test this in varying conditions in order to optimize each specific process.

One skilled in the art will appreciate that the selection of a suitable material for forming the biocompatible fibres of the present invention depends on several factors. These factors include in vivo mechanical performance; cell response to the material in terms of cell attachment, proliferation, migration and differentiation; biocompatibility; and optionally, bioabsorption (or bio-degradation) kinetics. Other relevant factors include the chemical composition, spatial distribution of the constituents, the molecular weight of the polymer, and the degree of crystallinity.

The fibres of the scaffold can be formed from a biocompatible polymer. A variety of biocompatible polymers can be used to make the fibres according to the present invention including synthetic polymers, natural polymers or combinations thereof. As used herein the term "synthetic polymer" refers to polymers that are not found in nature, even if the polymers are made from naturally occurring biomaterials. The term "natural polymer" refers to polymers that are naturally occurring. In embodiments where the fibres of the scaffold include at least one synthetic polymer, suitable biocompatible synthetic polymers can include polymers selected from the group consisting of aliphatic polyesters, poly-(amino acids), copoly(ether-esters), polyalkylene oxalates, polyamides, tyrosine derived polycarbonates, poly(iminocarbonates), polyorthoesters, polyoxaesters, polyamidoesters, polyoxaesters containing amine groups, poly(anhydrides), polyphosphazenes, polyurethanes, poly(ether urethanes), poly(ester urethanes), poly(propylene fumarate), poly-(hydroxyalkanoate) and blends thereof. Suitable synthetic polymers for use in the present invention can also include biosynthetic polymers based on sequences found in collagen, elastin, thrombin, silk, keratin, fibronectin, starches, poly(amino acid), gelatin, alginate, pectin, fibrin, oxidized cellulose, chitin, chitosan, tropoelastin, hyaluronic acid, ribonucleic acids, deoxyribonucleic acids, polypeptides, proteins, polysaccharides, polynucleotides and combinations thereof.

For the purpose of this invention aliphatic polyesters include, but are not limited to, homopolymers and copolymers of lactide (which includes lactic acid, D-, L- and meso lactide); glycolide (including glycolic acid); ε-caprolactone; p-dioxanone (1,4-dioxan-2-one); trimethylene carbonate (1,3- dioxan-2-one); alkyl derivatives of trimethylene carbonate; δ-valerolactone; β-butyrolactone; γ-butyrolactone; ε-decalactone; hydroxybutyrate; hydroxyvalerate; 1,4-dioxepan-2-one (including its dimer 1,5,8,12- tetraoxacyclotetradecane-7, 14-dione); 1,5- dioxepan-2-one; 6,6-dimethyl-1,4- dioxan-2-one; 2,5-diketomorpholine; pivalolactone; α,α diethylpropiolactone; ethylene carbonate; ethylene oxalate; 3-methyl-1,4- dioxane-2,5-dione; 3,3-diethyl-1,4-dioxan- 2,5-dione; 6,6-dimethyl-dioxepan-2-one; 6,8-dioxabicycloctane-7-one and polymer blends thereof.

Additional exemplary polymer or polymer blends include, by non-limiting example, a polydioxanone, a polyhydroxybutyrate-co-hydroxyvalerate, polyorthocarbonate, a polyaminocarbonate, and a polytrimethylene carbonate. Aliphatic polyesters used in the present invention can be homopolymers or copolymers (random, block, segmented, tapered blocks, graft, triblock, etc.) having a linear, branched or star structure. Poly(iminocarbonates), for the purpose of this invention, are understood to include those polymers as described by Kemnitzer and Kohn, in the Handbook of Biodegradable Polymers, edited by Domb, et. al., Hardwood Academic Press, pp. 251-272 (1997). Copoly(ether-esters), for the purpose of this invention, are understood to include those copolyester-ethers as described in the Journal of Biomaterials Research, Vol. 22, pages 993-1009, 1988 by Cohn and Younes, and in Polymer Preprints (ACS Division of Polymer Chemistry), Vol. 30(1), page 498, 1989 by Cohn (e.g., PEO/PLA). Polyalkylene oxalates, for the purpose of this invention, include those disclosed in US-4208511, US-4141087, US-4130639, US 4140678, US-4105034 and US-4205399. Polyphosphazenes, co-, ter- and higher order mixed monomer based polymers made from L-lactide, D, L-lactide, lactic acid, glycolide, glycolic acid, para-dioxanone, trimethylene carbonate and E-caprolactone such as are described by Allcock in The Encyclopedia of Polymer Science, Vol. 13, pages 31- 41, Wiley Intersciences, John Wiley & Sons, 1988 and by Vandorpe, et al in the Handbook of Biodegradable Polymers, edited by Domb, et al., Hardwood Academic Press, pp. 161-182 (1997). Polyanhydrides include those derived from diacids of the form:

HOOC--C₆H₄--O-- (CH₂)m--O--C₆H₄--COOH,

where "m" is an integer in the range of from 2 to 8, and copolymers thereof with aliphatic alpha-omega diacids of up to 12 carbons. Polyoxaesters, polyoxaamides and polyoxaesters containing amines and/or amido groups are disclosed in US-5464929, US-5595751, US-5597579, US-5607687, US-5618552, US-5620698, US-5645850, US-5648088, US-5 698213, US-5700583 and US-5859150. Polyorthoesters such as those described by Heller in Handbook of Biodegradable Polymers, edited by Domb, et al., Hardwood Academic Press, pp. 99-118 (1997).

As used herein, the term "glycolide" is understood to include polyglycolic acid. Further, the term "lactide" is understood to include L- lactide, D-lactide, blends thereof, and lactic acid polymers and copolymers. Elastomeric copolymers are also particularly useful in the present invention, including, but not limited to, elastomeric copolymers of α-caprolactone and glycolide (including polyglycolic acid) with a mole ratio of ε-caprolactone to glycolide of from about 35:65 to about 65:35, more preferably from 45:55 to 35:65; elastomeric copolymers of ε-caprolactone and lactide (including L-lactide, D-lactide, blends thereof, and lactic acid polymers and copolymers) where the mole ratio of ε-caprolactone to lactide is from about 35:65 to about 65:35 and more preferably from 45:55 to 35:65 or from about 95:5 to about 85:15; elastomeric copolymers of p-dioxanone (1,4-dioxan-2-one) and lactide (including L-lactide, D-lactide, blends thereof, and lactic acid polymers and copolymers) where the mole ratio of p-dioxanone to lactide is from about 40:60 to about 60:40; elastomeric copolymers of ε-caprolactone and p-dioxanone where the mole ratio of ε-caprolactone to p-dioxanone is from about from 30:70 to about 70:30; elastomeric copolymers of p- dioxanone and trimethylene carbonate where the mole ratio of p-dioxanone to trimethylene carbonate is from about 30:70 to about 70:30; elastomeric copolymers of trimethylene carbonate and glycolide (including polyglycolic acid) where the mole ratio of trimethylene carbonate to glycolide is from about 30:70 to about 70:30; elastomeric copolymers of trimethylene carbonate and lactide (including L-lactide, D-lactide, blends thereof, and lactic acid polymers and copolymers) where the mole ratio of trimethylene carbonate to lactide is from about 30:70 to about 70:30; and blends thereof. Examples of suitable biocompatible elastomers are disclosed in US-4045418.

In one embodiment, the elastomer is a copolymer of 35:65 ε-caprolactone and glycolide, formed in a dioxane solvent. In another embodiment, the elastomer is a copolymer of 40:60 ε-caprolactone and lactide. In yet another embodiment, the elastomer is a 50:50 blend of a 35:65 copolymer of ε-caprolactone and glycolide and 40:60 copolymer of ε-caprolactone and lactide.

The fibres of the present invention can, optionally, be formed from a bioresorbable or bioabsorbable material that has the ability to resorb in a timely fashion in the body environment. The differences in the absorption time under *in vivo* conditions can also be the basis for combining two different copolymers when forming the fibres of the present invention. For example, a copolymer of 35:65 ε-caprolactone and glycolide (a relatively fast absorbing polymer) can be blended with 40:60 ε-caprolactone and L-lactide copolymer (a relatively slow absorbing polymer) to form a biocompatible fibre. Depending upon the processing technique used, the two constituents can be either randomly inter-connected bicontinuous phases, or the constituents could have a gradient-like architecture with a well integrated interface between the two constituent layers.

In one embodiment, it is desirable to use polymer blends to form fibres which transition from one composition to another composition in a gradient-like architecture. Scaffolds having this gradient-like architecture are particularly advantageous in tissue engineering applications to repair or regenerate the structure of naturally occurring tissue such as cartilage (articular, meniscal, septal, tracheal, auricular, costal, etc.), tendon, ligament, nerve, esophagus, skin, bone, and vascular tissue. Other polymer blends may be used for similar gradient effects, or to provide different gradients (e.g., different absorption profiles, stress response profiles, or different degrees of elasticity). For example, such design features can establish a concentration gradient for the suspension of minced tissue associated with the prosthesis of the present invention, such that a higher concentration of the tissue fragments is present in one region of the scaffold (e.g., an interior portion) than in another region (e.g., outer portions).

The gradient-like transition between compositions can also be oriented in the radial direction of the fibres. For example, some of the fibres of the scaffold may be co-extruded to produce a fibre with a sheath/core construction. Such fibres are comprised of a sheath of biodegradable polymer that surrounds one or more cores comprised of another biodegradable polymer. Fibres with a fast-absorbing sheath surrounding a slower-absorbing core may be desirable for extended support.

Although not all named polymers may be extruded using melt-blowing technology, some of these materials may serve as the take up object or may be layered into the final composite.

To facilitate cell growth and infiltration a porous structure is of particular significance to any nonwoven matrix. Each nonwoven may be characterized by the pore size and porosity of the final construct. Desired pore sizes may range from 10 to 350 µm. In a preferred embodiment the pore size in the medical product can be 20 to 200 µm. The desired final melt-blown fibrous composite should have a porosity of 50 to 99% for optimal cell adhesion and growth.

To manufacture the scaffolds described above a porogen with a diameter in the range of 20 µm to 2 mm is suggested. These porogens may be made from glucose, sucrose, NaCl or any other suitable material used in particle leeching. One skilled in the art will appreciate that the selection of a suitable porogen for forming the porosity and interconnectedness of the present invention depends on several factors. These factors include porogen size, weight, melting temperature and chemical composition. Other relevant factors include the spatial distribution of the porogen, the weight of the porogen, and the quantitative mass flow of the porogen into the extrusion stream.

### Example 2. Hollow Matrix Fabricated by Melt-Blown Technology

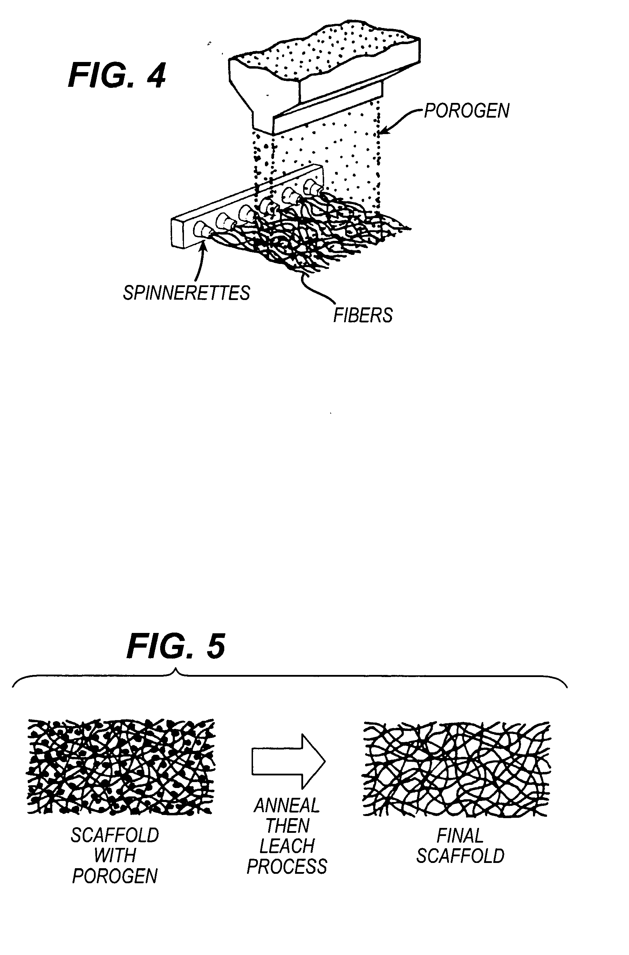

A biopolymer suitable for fibre formation is forced through a spinnerette containing a small aperture. A die with numerous spinnerettes extrudes the polymer onto a rotating three-dimensional, collapsible object, which serves as the take-up or collecting surface. See Figure 2. The extruded polymer is simultaneously subjected to heated air, forced at a very high velocity by cooperating gas orifices positioned at slight angles to the direction of extrusion to cause attenuation and elongation of extruded molten fibres. See Figure 1. Although the spinnerettes are fixed facing a single direction, the turbulent forces caused by the pressurized air cause the fibres to randomly arrange and entangle themselves during which the fibres also bond to each other so as to form a coherent mass. Rotating the collapsible object onto which the polymers are extruded allows for a substantially even polymer layer. Also during extrusion of the polymer fibres, a salt porogen is dusted onto the molten fibres. See Figure 4. Once the porogen-fibre composite has annealed, the entire construct is submerged in water so that the porogen dissolves whereby a non-woven web of flexible polymer fibres remains. See Figure 5. A three-dimensional, collapsible object is used to create a seamless, hollow, three-dimensional polymer web that is both biocompatible and biodegradable upon collapsing.

## Claims

1. A tissue growth device comprising a biocompatible, biodegradable scaffold capable of integral cell growth that forms a hollow chamber.

2. The device of claim 1 in which the scaffold is produced by melt-blowing a web of flexible polymer fibres in the presence of a porogen.

3. The device of claim 2 in which the melt-blowing comprises distributing molten polymer resin onto a moveable collapsible object to create a seamless, three-dimensional shape.

4. The device of claim 2 in which the flexible polymer fibres further comprise an inner core of a slower degrading polymer and an outer sheath of a faster degrading polymer.

5. The device of claim 4 in which the inner and outer polymer layers further comprise a transition layer between the polymer layers, the transition layer comprising a gradient.

6. The device of claim 2 in which the porogen is selected from the group consisting of glucose, sucrose, gelatin, and salt.

7. The device of claim 2 in which the porogen is sized from about 20 microns to about 2 mm.

8. The device of claim 1 which includes a pharmaceutical agent.

9. The device of claim 8 in which the pharmaceutical agent is selected from the group consisting of: antibiotics, antiviral agents, chemotherapeutic agents, anti-rejection agents, analgesics, anti-inflammatory agents, hormones, steroids, growth factors, proteins, polysaccharides, glycoproteins, and lipoproteins.

10. The device of claim 1 which includes a subdivision in the hollow chamber.

11. The device of claim 1 which includes a plurality of hollow chambers.

12. A method of making a hollow tissue growth device comprising the steps of:
a. providing a movable collapsible object, and
b. providing a molten stream of polymer fibres, and
c. adding a porogen to the molten stream of polymer fibres, and
d. melt-blowing the molten stream of polymer fibres onto the collapsible object, and
e. removing the collapsible object, and
f. removing the porogen.

13. The method of claim 12 in which the molten stream of polymer fibres further comprises a first molten stream of slow degrading fibres and a second molten stream of fast degrading fibres.

14. The method of claim 13 which includes varying the proportions of the first and second molten streams of polymer fibres to provide a gradient.

15. A tissue generated using the device of claim 1.

16. The tissue of claim 15 in which the tissue is bladder tissue.
